# EUROPÄISCHE PATENTSCHRIFT

(11) **EP 0 577 990 B1**
(45) Veröffentlichungstag und Bekanntmachung des Hinweises auf die Patenterteilung: **04.03.1998**
(21) Anmeldenummer: 93109247.2
(22) Anmeldetag: 09.06.1993
(51) Int. Cl.: C07C 29/141, B01J 8/06, B01J 10/00, B01J 12/00, F28F 9/22

(54) **Verfahren zur katalytischen Hydrierung organischer Verbindungen in der Gasphase und Wärmetauscher zur Durchführung dieses Verfahrens**
Method of catalytically hydrogenating organic compounds in gasphase and heat exchanger used in the process
Procédé d'hydrogénation catalytique de composés organiques et échangeur de chaleur utilisé dans dans le procédé

(30) Priorität: 25.06.1992 DE 4220783
(43) Veröffentlichungstag der Anmeldung: 12.01.1994
(73) Patentinhaber: HOECHST AKTIENGESELLSCHAFT, 65926 Frankfurt am Main (DE)
(72) Erfinder: Höfs, Wolfgang, Dipl.-Ing., D-4200 Oberhausen (DE); Müller, Thomas, Dipl.-Ing., D-4220 Dinslaken (DE)

(56) Entgegenhaltungen:
- EP-A- 0 073 129
- EP-A- 0 215 563
- WO-A-91/01961

## Beschreibung

Die Erfindung betrifft die katalytische Hydrierung organischer Verbindungen, insbesondere die Hydrierung von Aldehyden und Oxialdehyden zu ein- oder mehrwertigen Alkoholen in der Gasphase, und ein Heißgaswärmeaustauscher zur Durchführung dieses Verfahrens.

Die Hydrierung organischer Verbindungen wie gesättigte oder ungesättigte Aldehyde oder Oxialdehyde erfolgt in der industriellen Praxis im allgemeinen kontinuierlich mit, bezogen auf Aldehyd, überschüssigem Wasserstoff. Nicht umgesetzter Wasserstoff wird in den Hydrierreaktor rezirkuliert (Kreisgas), üblicherweise, nachdem ein Teil des mit Inerten und Ballaststoffen beladenen Wasserstoffs abgetrennt und diese abgetrennte sowie die verbrauchte Menge durch Frischgas ersetzt wurde.

Der Gesamtprozeß verläuft gewöhnlich in folgenden Einzelschritten, wobei selbstverständlich Variationen oder Anpassungen an individuelle Erfordernisse möglich sind: Wasserstoff in Form von Frischgas und Kreisgas wird zur Überwindung des im System bestehenden Druckverlustes komprimiert. Gleichzeitig, jedoch getrennt voneinander, führt man den Wasserstoff mit dem zu hydrierenden Einsatzstoff einem Wärmeaustauscher zu. Hier werden die Reaktanten durch das Reaktionsprodukt vorgewärmt und, soweit sie nicht gasförmig vorliegen` zumindest teilweise verdampft, während sich das Reaktionsprodukt abkühlt und höhersiedende Anteile auskondensieren. Die nichtverdampften Reaktanten können in einem nachfolgenden Wärmeaustauscher bis auf einen geringen Flüssiganteil, der abgezogen wird, in die Gasphase überführt und schließlich in einem Überhitzer auf die Reaktionstemperatur aufgeheizt, dem Reaktor zugeführt werden. Hier erfolgt bei annähernd konstanter Temperatur die Hydrierung, die dabei freiwerdende Wärme wird zur Dampferzeugung genutzt. Das heiße Produkt gelangt in den bereits genannten Wärmeaustauscher und darauf in einen Kondensator. Ein etwa in den vorgezeichneten Stufen ablaufender Hydrierprozeß ist z.B. in Ullmanns Encyklopädie der technischen Chemie, 3. Auflage (1953), S. 778, 779 für die Hydrierung von Crotonaldehyd zu Butanol beschrieben.

In der Praxis kommt es im wesentlichen darauf an, den Wärmehaushalt des gesamten Prozesses optimal zu gestalten, d.h. einen möglichst hohen energetischen Wirkungsgrad zu erzielen. Darüber hinaus soll vermieden werden, daß die Aktivität des Katalysators durch Fremdstoffe, die mit den gasförmigen Reaktionspartnern in den Hydrierreaktor eingeschleppt werden, geschädigt wird. Hierzu ist es u.a. erforderlich, Nebenprodukte aus dem in den Hydrierreaktor eintretenden Produktstrom möglichst vollständig abzutrennen.

Es hat sich gezeigt, daß höhere Druckverluste in der Hydrieranlage zu einem Anstieg der Gasaustrittstemperatur am Verdichter führen und als Folge davon die Wärmeübertragung am Wärmeaustauscher verschlechtern. Weiterhin verbessert bekanntermaßen eine liegende Anordnung des Wärmeaustauschers zwar die Kondensation der Reaktionsprodukte, sie setzt aber ebenfalls den Wärmeübergang auf der Rohrseite des Wärmeaustauschers entscheidend herab.

Es bestand daher die Aufgabe, ein Verfahren zur Hydrierung organischer Verbindungen zu entwickeln, das die aufgezeigten Nachteile vermeidet.

Die Erfindung besteht in einem Verfahren zur katalytischen Hydrierung organischer Verbindungen in der Gasphase. Es ist dadurch gekennzeichnet, daß das einen Hydrierreaktor (4) verlassende Kreisgas einschließlich dem Reaktionsprodukt ohne vorherige Kühlung in einem Kompressor (5) auf einen zur Überwindung des Druckverlustes in der Anlage ausreichenden Druck verdichtet wird, in einem Überhitzer (3) die Einsatzstoffe auf die Reaktionstemperatur erhitzt, darauf in einem Heißgaswärmeaustauscher (1) im Gegenstrom zu den Reaktanten geführt und dadurch weiter abgekühlt wird, so daß das Reaktionsprodukt weitgehend auskondensiert und nach Zusatz von Frischgas und Einsatzstoff über eine Reinigungskolonne (2) wieder dem Hydrierreaktor (4) zugeleitet wird.

Das neue Verfahren gewährleistet eine hervorragende Nutzung der dem System zugeführten und im System entwickelten Energien und stellt überdies eine scharfe Trennung der im Reaktionsprodukt enthaltenen Wertstoffe und Nebenprodukte vom Kreisgas sicher, so daß der Katalysator insbesondere durch die Ablagerung höher siedender Stoffe nicht belastet wird.

Die Verdichtung des Reaktionsprodukt enthaltenden Kreisgases nach Verlassen des Reaktors vermehrt seinen Wärmeinhalt um die Kompressionswärme. Die thermische Energie wird dazu genutzt, die Einsatzstoffe, z.B. Aldehyd und Wasserstoff, in einem Überhitzer auf die Reaktionstemperatur zu erhitzen. Durch Einhaltung einer sehr geringen Verweilzeit wird dabei vermieden, daß das Reaktionsprodukt gegebenenfalls thermisch geschädigt wird. Das aus dem Überhitzer kommende Gas gelangt in einen Heißgaswärmeaustauscher, der als Fallfilmverdampfer ausgebildet ist. In ihm wird es durch im Gegenstrom geführte Reaktanten, die verdampfen, nachhaltig abgekühlt mit der Folge, daß Hydrierprodukt auskondensiert. Eine weitere Herabsetzung der Temperatur des Gasstromes erfolgt in einem nachgeschalteten Kondensator, in dem restliches Hydrierprodukt abgeschieden wird. Um den Inerten- und Ballaststoffgehalt zu begrenzen, wird ein kleiner Teil des Kreisgases als Abgas aus dem Gasstrom abgetrennt. Der weitaus größere Teil des Kreisgases, im wesentlichen Wasserstoff und neben Inerten und Ballaststoffen geringe Anteile nichtkondensierbarer Reaktionsprodukte, wird in den Heißgaswärmeaustauscher zurückgeführt, mit ebenfalls eingeleitetem Einsatzmaterial gemischt und anschließend einer Reinigungskolonne aufgegeben. Hier wird das Einsatzgemisch unter Verwendung mehrerer Gleichgewichtsstufen rektifiziert. In einem nachgeschalteten Dephlegmator wird die Kondensation höhersiedender Anteile des Gasstromes vervollständigt. Die Verwendung einer Reinigungskolonne im Rahmen des erfindungsgemäßen Prozesses stellt sicher, daß Anteile, die höher als das Hydrierprodukt sieden, nicht in den Reaktor und damit auch nicht auf den Katalysator gelangen. Frischer Wasserstoff, der den bei der Hydrierung verbrauchten ersetzt, wird in den Reboiler der Reinigungskolonne eingeleitet, er senkt dort die Siedetemperatur zur Schonung des Produktes, sodann dem Überhitzer und anschließend dem Hydrierreaktor zugeleitet.
Nach einer bevorzugten Ausführungsform des erfindungsgemäßen Verfahrens verwendet man als Heißgaswärmeaustauscher einen in besonderer Weise gestalteten Fallfilmverdampfer, in dem die Führung der Stoffströme im Gegenstrom erfolgt, eine für diesen Apparatetyp unübliche Betriebsweise. Daher sind besondere Maßnahmen notwendig, den Wärmeübergang auf der Mantelseite der Austauscherrohre optimal zu gestalten. Erfindungsgemäß sind die Rohre auf der Außenseite mit gewendelten Rillen oder Drahtwickeln versehen. Auf Grund ihrer Oberflächenspannung sammelt sich die Flüssigkeit bevorzugt an den Rillen oder Drahtwickeln, so daß ausreichend freie Austauschfläche zur Kondensation bereitsteht. Die Umlenkbleche an den Wärmeaustauscherrohren sind als Kondensatsammler ausgestaltet. Jedes Blech besitzt einen Abfluß über ein Tauchrohr, das in das Kondensat am Fuße des Wärmeaustauschers eintaucht.

In den folgenden Abbildungen werden das neue Verfahren und der zu seiner Ausübung eingesetzte Heißgaswärmeaustauscher erläutert. Abbildung 1 gibt eine schematische Darstellung des neuen Verfahrens, Abbildung 2 eine Ausführungsform des im Rahmen der Erfindung bevorzugt eingesetzten Heißgaswärmeaustauschers und Abbildung 3 im Detail die Gestaltung der Wärmeaustauschrohre des Heißgaswärmeaustauschers wieder.

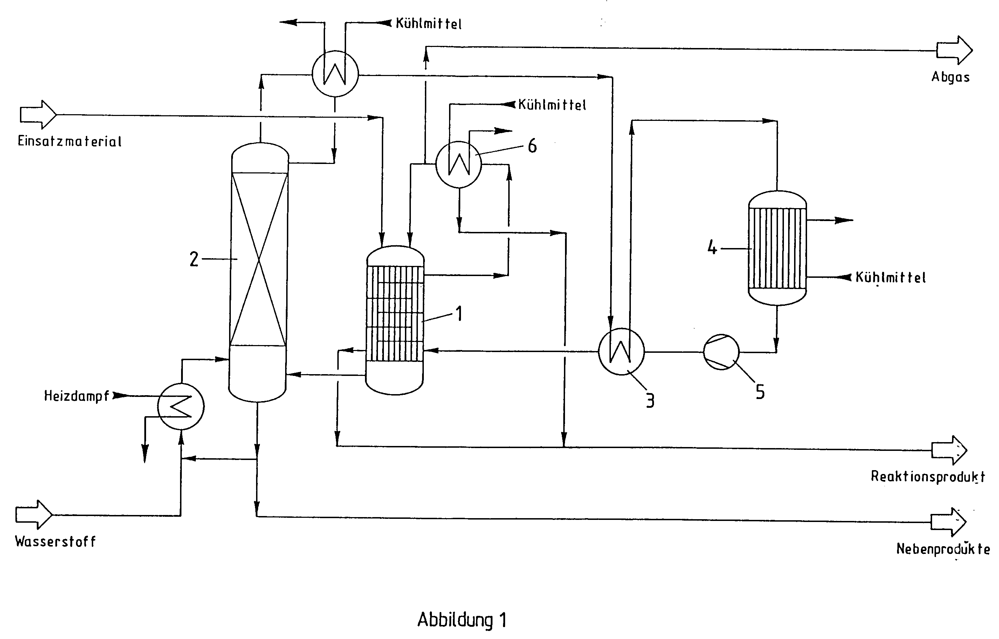

In dem Verfahren nach Abbildung 1 wird das zu hydrierende Einsatzmaterial in einem Heißgaswärmeaustauscher 1 durch im Gegenstrom geführtes Reaktionsprodukt aufgeheizt und zusammen mit rückgeführtem und gesondert eingespeistem frischen Wasserstoff über eine Reinigungskolonne 2 einem Überhitzer 3 zugeführt. Das in dem Überhitzer auf Reaktionstemperaturen erhitzte Gemisch wird in einem Reaktor 4 umgesetzt. Das Reaktionsprodukt wird in einem Kompressor 5 zur Überwindung des Druckverlustes in der Anlage komprimiert und nach Abkühlung im Überhitzer 3 dem Heißgaswärmeaustauscher 1 zugeführt, in dem unter Aufheizen des Einsatzmaterials das Reaktionsprodukt weiter abgekühlt wird und durch Kondensation eine Trennung in gasförmige und flüssige Phase erfolgt. Die gasförmige Phase wird in einen Kondensator 6 geleitet, in dem weitere kondensierbare Anteile der den Heißgaswärmeaustauscher verlassenden Gasphase abgetrennt werden. Das Abgas des Kondensators wird in den Heißgaswärmeaustauscher zurückgeführt.

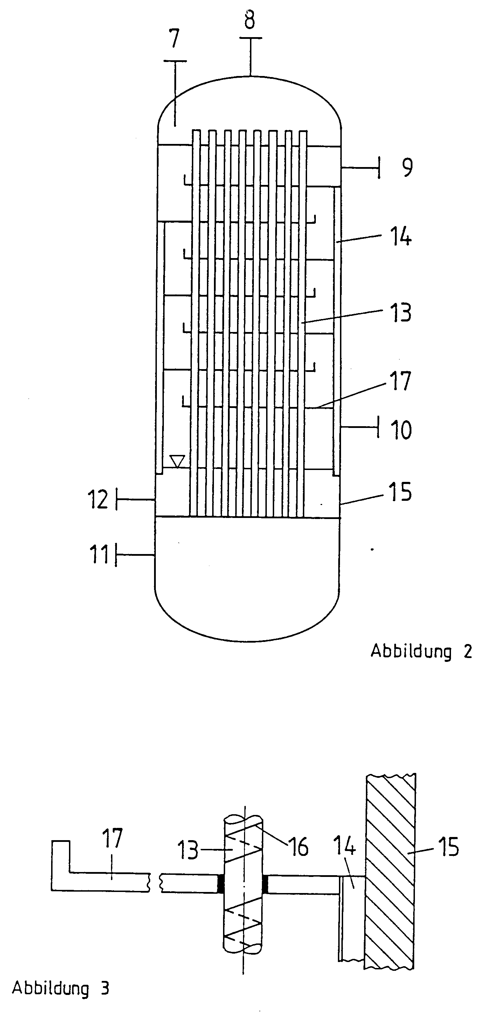

Bei der in Abbildung 2 dargestellten Ausführungsform eines Heißgaswärmeaustauschers tritt heißes Reaktionsprodukt über einen Stutzen 10 in den Apparat ein. Er wird mit Hilfe von Umlenkblechen 17 im Kreuzgegenstrom den kalten, flüssigen und gasförmigen Einsatzstoffen entgegengeführt, die über einen Stutzen 7 bzw. einen Stutzen 8 eingeleitet werden. Der flüssige Einsatzstoff bildet unter der Wirkung bekannter Verteilereinrichtungen an den Innenseiten von Rohren 13 einen dünnen Film.

Das am Taupunkt befindliche heiße Reaktionsprodukt überträgt seinen Wärmeinhalt auf die kalten Einsatzstoffe und kondensiert dabei teilweise, während der flüssige Anteil der Einsatzstoffe verdampft. Die aufgeheizten Einsatzstoffe verlassen den Apparat über einen Stutzen 11. Das abgekühlte Reaktionsprodukt wird gasförmig an einem Stutzen 9 und als Kondensat an einem Stutzen 12 abgezogen. Durch Einhaltung der beschriebenen Maßnahmen kann erreicht werden, daß die Austrittstemperatur der Einsatzstoffe höher ist als die Austrittstemperatur der Reaktionsprodukte. Mit Hilfe eines Heißgaswärmeaustauschers der in Abbildung 2 dargestellten Art werden kurze Kondensationstrecken und dünne Kondensationsfilme gewährleistet, die zu einem guten Wärmeübergang beitragen. Dazu ist eine Ableitung des Kondensates von dem auch als Sammelvorlage dienenden Umlenkblechen 17 notwendig; sie erfolgt mit Tauchrohren 14.

Die Abbildung 3 zeigt detailliert den Aufbau eines Umlenkbleches 17 mit einer senkrecht stehenden Außenseite zur Sammlung des Kondensates und seine Abdichtung gegenüber dem Wärmeaustauscherrohr 13 und dem der Kondensatabführung dienenden Tauchrohr 14. Das Rohr 13 besitzt auf der Außenfläche Kondensatsammelhilfen 16, z.B. in Form von gewendelten Rillen oder Drahtwickeln, die bekanntermaßen die freie Kondensationsfläche erhöhen.

## Patentansprüche

1. Verfahren zur katalytischen Hydrierung organischer Verbindungen in der Gasphase, dadurch gekennzeichnet, daß das einen Hydrierreaktor (4) verlassende Kreisgas einschließlich dem Reaktionsprodukt ohne vorherige Kühlung in einem Kompressor (5) auf einen zur Überwindung des Druckverlustes in der Anlage ausreichenden Druck verdichtet wird, in einem Überhitzer (3) die Einsatzstoffe auf die Reaktionstemperatur erhitzt, darauf in einem Heißgaswärmeaustauscher (1) im Gegenstrom zu den Reaktanten geführt und dadurch weiter abgekühlt wird, so daß das Reaktionsprodukt weitgehend auskondensiert und nach Zusatz von Frischgas und Einsatzstoff über eine Reinigungskolonne (2) wieder dem Hydrierreaktor (4) zugeleitet wird.

2. Verfahren nach Anspruch 1 dadurch gekennzeichnet, daß die organischen Verbindungen gesättigte oder ungesättigte Aldehyde oder Oxialdehyde sind.

3. Heißgaswärmeaustauscher zur Durchführung des Verfahrens nach Anspruch 1 gekennzeichnet durch mit Kondensatsammelhilfen (16) versehene Rohre (13), als Kondensatsammler ausgestaltete Umlenkbleche (17) an den Rohren (13) und der Kondensatabführung dienende Tauchrohre (14).

4. Heißgaswärmeaustauscher nach Anspruch 3 dadurch gekennzeichnet, daß die Kondensatsammelhilfen (16) als gewendelte Rillen oder Drahtwickel ausgebildet sind.

## Claims

1. A process for the catalytic hydrogenation of organic compounds in the gas phase, which comprises a circulating gas, including the reaction product, leaving a hydrogenation reactor (4) being compressed without prior cooling in a compressor (5) to a pressure sufficient to overcome the pressure drop in the installation, heating the starting materials to the reaction temperature in a superheater (3), then being conducted in counter-current to the reactants in a hot-gas heat exchanger (1) and, as a result, cooled further, so that the reaction product substantially condenses out and, after addition of fresh gas and starting material, being returned via a purification column (2) to the hydrogenation reactor (4).

2. The process as claimed in claim 1, wherein the organic compounds are saturated or unsaturated aldehydes or oxyaldehydes.

3. A hot-gas heat exchanger for carrying out the process as claimed in claim 1, having tubes (13) furnished with condensate collection aids (16), deflection baffles (17) designed as condensate receivers on the tubes (13) and immersion pipes (14) serving for the condensate discharge.

4. The hot-gas heat exchanger as claimed in claim 3 wherein the condensate collection aids (16) are designed as spiral grooves or wire coils.

## Revendications

1. Procédé d'hydrogénation catalytique de composés organiques en phase gazeuse caractérisé en ce que le gaz de recyclage, y compris le produit de la réaction, qui quitte un réacteur d'hydrogénation (4) est comprimé sans refroidissement préalable dans un compresseur (5) à une pression suffisante pour vaincre la perte de pression dans l'installation, chauffe les substances de charge à la température de la réaction dans un surchauffeur (3) puis est introduit dans un échangeur de chaleur à gaz chauds (1) à contre-courant des corps réagissants et est de ce fait refroidi encore de sorte que le produit de la réaction est largement condensé et, après addition de gaz frais et de substance de charge, est renvoyé au réacteur d'hydrogénation (4) par l'intermédiaire d'une colonne d'épuration (2).

2. Procédé selon la revendication 1, caractérisé en ce que les composés organiques sont des aldéhydes ou des oxyaldéhydes saturés ou insaturés.

3. Echangeur de chaleur à gaz chauds pour la mise en oeuvre du procédé selon la revendication 1, caractérisé par des tubes (13) munis d'auxiliaires de collecte de condensat (16), de déflecteurs (17) sous forme de collecteurs de condensat au niveau des tubes (13) et de tubes plongeurs (14) servant à l'évacuation du condensat.

4. Echangeur de chaleur à gaz chauds selon la revendication 3, caractérisé en ce que les auxiliaires de collecte de condensat (16) sont sous forme de rainures en spirale ou d'enroulements de fil.
